**Europäisches Patentamt**

**European Patent Office**

**Office européen des brevets**

(11) Veröffentlichungsnummer: **0 306 985 B1**

# EUROPÄISCHE PATENTSCHRIFT

(45) Veröffentlichungstag der Patentschrift: **04.12.91**

(51) Int. Cl.⁵: **C12N 1/20**, C12N 15/01, C12P 21/02

(21) Anmeldenummer: 88114806.8

(22) Anmeldetag: 09.09.88

(54) **Bakteriorhodopsin-Modifikationen und Herstellungsverfahren.**

(30) Priorität: 10.09.87 DE 3730424

(43) Veröffentlichungstag der Anmeldung:
15.03.89 Patentblatt 89/11

(45) Bekanntmachung des Hinweises auf die
Patenterteilung:
04.12.91 Patentblatt 91/49

(84) Benannte Vertragsstaaten:
**CH DE FR GB LI NL**

(56) Entgegenhaltungen:

**ANNALES DE MICROBIOLOGIE, Band 134B, Nr. 1, 1983, Seiten 137-150; D. OESTERHELT et al.: "Phototrophic growth of halobacteria and its use for isolation of photosynthetically-deficient mutants"**

**THE JOURNAL OF BIOLOGICAL CHEMISTRY, Band 262, Nr. 19, 5. Juli 1987, Seiten 9277-9284; N.R. HACKETT et al.: "Structure-Function studies on bacteriorhodopsin"**

**CHEMICAL ABSTRACTS, Band 105, Nr. 17, 27**

**Oktober 1986, Seite 201, Zusammenfassung Nr. 147529z, Columbus, Ohio, US; M.A. GILLES-GONZALEZ et al.: "Methods for mutagenesis of the bacterioopsin gene", & METHODS ENZYMOL. 1986, 125 (Biomembranes, Pt. M), 190-214**

(73) Patentinhaber: **Max-Planck-Gesellschaft zur Förderung der Wissenschaften e.V.
Bunsenstrasse 10
W-3400 Göttingen(DE)**

(72) Erfinder: **Oesterhelt, Dieter, Dr.
Werdenfelsstrasse 17
W-8000 München 70(DE)**
Erfinder: **Soppa, Jörg
Alpspitzstrasse 11
W-8000 München 70(DE)**
Erfinder: **Krippahl, Günther
Am Klopferspitz 18
W-8033 Martinsried(DE)**

(74) Vertreter: **Wagner, Helga, Dr.
c/o Wacker-Chemie GmbH Prinzregentenstrasse 22
W-8000 München 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bakteriorhodopsin wird von Halobacterium wie Halobacterium halobium produziert und stellt ein Protein dar, dessen reiner Proteinanteil (das Bakterioopsin) einen Chromophor (das Retinal) trägt. Bakteriorhodopsin, das aus dem Wildtyp der genannten Mikroorganismen gewonnen worden ist, besitzt maximale Absorption bei 560 nm. Die Lebenszeit eines Zwischenproduktes beträgt 10 msec. Aufgrund der lichtelektrischen Eigenschaften von Bakteriorhodopsin ist es seit langem erwünscht, diesen Stoff für Biocomputer zu verwenden; vgl. beispielsweise Biosystems, 19 (1986) 223 - 236. Bisher ist es jedoch noch nicht gelungen, Bakteriorhodopsin-Modifikationen herzustellen, die eine verlängerte Lebenszeit eines Zwischenprodukts oder des langlebigsten Zwischenprodukts bieten oder eine andere Farbe als Bakteriorhodopsin des Wildtyps besitzen.

Es ist bereits ein Verfahren angesprochen worden, nach dem Halobacterium-halobium-Mutanten erhalten werden können; vgl. Ann. Microbiol. (Inst. Pasteur), 134 B (1983) 137 - 150. Nach diesem bekannten Verfahren kann man Bakteriorhodopsin-Modifikationen dadurch gewinnen, daß man

(a) Halobacterium-halobium mit funktionell intaktem Bakteriorhodopsin einer Mutagenese in an sich bekannter Weise unterwirft,

(b) das erhaltene Zellgemisch unter anaeroben Bedingungen in Gegenwart von BDU in an sich bekannter Weise solange kultiviert, bis eine ohne BDU kultivierte Kontrollkultur die späte logarithmische oder stationäre Phase erreicht, und

(c) das in Gegenwart von BDU kultivierte Zellgemisch mit Licht einer Wellenlänge im Bereich von 250 bis 500 nm belichtet, um nicht mutierte Zellen abzutöten. Nach diesem Stand der Technik ist es auch bekannt, daß retinalnegative Mutanten dann wieder wachsen, wenn man Retinal zum Kulturmedium zugibt; loc. cit. 147/148. Man könnte nach diesem Stand der Technik also auch noch daran denken, zusätzlich zu nicht mutierten Zellen retinalnegative Mutanten abzutöten. ES bestünden jedoch auch dann praktisch keine Aussichten,

- zu Bakteriorhodopsin-Modifikationen mit einer gegenüber dem Bakteriorhodopsin des Wildtyps verlängerten Lebenszeit eines Zwischenprodukts bzw. des langlebigsten Zwischenproduktes oder
- zu Bakteriorhodopsin-Modifikationen mit einer von der Farbe des Wildtyps (560 nm) abweichenden Farbe zu gelangen. Auch wert man unterstellt, daß das Zellgemisch brauchbare Mutanten umfaßt, so würden daneben noch nicht abgetötete Wildtyp-Zellen sowie Mutanten vorliegen, die Bakteriorhodopsin (nur noch) schwach exprimieren. Diese zuletzt genannten Mutanten würden sich auf Plattenkulturen nicht von gewünschten Mutanten unterscheiden, da sie gleichfalls anaerob nicht wachsen und sich damit nicht klonieren lassen würden; zur Klonierung von Halobacterium-halobium auf Plattenkulturen vgl. beispielsweise Proc. Natl. Acad. Sci. USA, 77 (1980) 3821 - 3825 und Methods in Enzymology, 88 (1982) 417 - 420.

Auch unabhängige Versuche, funktionslose Bakteriorhodopsin-Modifikationen zu erzeugen und in Escherichia coli zu exprimieren, sind bisher erfolglos geblieben; vgl. beispielsweise Ann. Rep. MIT, (1985) 190 -220.

Aufgabe der Erfindung ist es, Bakteriorhodopsin-Modifikationen

- mit einer gegenüber dem Bakteriorhodopsin eines Halo-bacterium-Wildtyps verlängerten Lebenszeit eines Zwischenprodukts bzw. des langlebigsten Zwischenproduktes sowie Gewinnungsverfahren vorzusehen.

Eine weitere Aufgabe der Erfindung ist es, Halobacterienstämme nach Anspruch 1 sowie ein Verfahren zu ihrer Gewinnung vorzusehen.

Dazu werden gemäß einer Ausführungsform der Erfindung Halobacterium-Stämme vorgesehen, die Bakteriorhodopsin-Modifikationen

- mit einer gegenüber dem Bakteriorhodopsin des Wildtyps verlängerten Lebenszeit eines Zwischenprodukts bzw. des langlebigsten Zwischenproduktes von mehr als 10 msec und/oder
- mit einer Farbe im Bereich von 400 bis 650 nm produzieren, wobei die Farbe des Wildtyps (560 nm) ausgenommen ist.

Zur Herstellung der erfindungsgemäßen Halobacterium-Stämme wird ein Verfahren vorgesehen, bei dem man

(a) Halobacterium spec. mit funktionell intaktem Bakteriorhodopsin einer Mutagenese in an sich bekannter Weise unterwirft,

(b) das erhaltene Zellgemisch unter anaeroben Bedingungen in Gegenwart von 5-Brom-2'-deoxyuridin (BDU) in an sich bekannter Weise so lange kultiviert, bis eine ohne BDU kultivierte Kontrollkultur die späte logarithmische oder stationäre Phase erreicht,

(c) das in Gegenwart von BDU kultivierte Zellgemisch mit Licht einer Wellenlänge im Bereich von 300 bis 550 nm in an sich bekannter Weise belichtet, um nicht mutierte Zellen abzutöten, und

(d) gegebenenfalls die Stufen (b) und (c) wiederholt, wobei das Verfahren dadurch gekennzeichnet ist, daß man

(I) bei der Stufe (a) von einem Wildtyp mit einer Reversionsrate kleiner 1:10⁶ ausgeht,

(II) die Belichtung der Stufe (c) mit 100 bis 200 mW/cm² 5 min bis 2 h lang durchführt,

(III) die Kultivierung der Stufe (b) in Gegenwart von Retinal durchführt oder mit dem aus Stufe (c) oder (d) erhaltenen Zellgemisch die Stufe (b) in Gegenwart von Retinal und danach die Stufe (c) und gegebenenfalls die Stufe (d) wiederholt, um retinalnegative Mutanten abzutöten,

(IV) das aus Stufe (III) erhaltene Zellgemisch aerob als Plattenkultur anzüchtet,

(V) die angezüchtete Plattenkultur anaerob weiterkultiviert,

(VI) alle Klone, die anaerob phototroph nicht mehr wachsen, wie Klone mit Bakterio-opsin-Modifikationen oder mit gestörter Bakterio-opsin-Synthese, mit Antikörpern gegen den C-Terminus der Bakterio-opsin-Modifikationen (bzw. Bakteriorhodopsin-Modifikationen) einem Kolonie-Screening in an sich bekannter Weise unterwirft und

(VII) Klone, die Bakteriorhodopsin-Modifikationen exprimieren, in an sich bekannter Weise gewinnt.

Gemäß einer weiteren Ausführungsform der Erfindung werden Bakteriorhodopsin-Modifikationen mit einer gegenüber dem Bakteriorhodopsin eines Halobacterium-Wildtyps verlängerten Lebenszeit eines Zwischenprodukts bzw. des langlebigsten Zwischenproduktes von mehr als 10 msec vorgesehen. Vorzugsweise liegt die Lebenszeit im Bereich von mehr als 10 msec bis 1 sec oder bis 1 min oder bis unendlich und insbesondere im Bereich von 0,5 sec bis unendlich.

Zur Herstellung der erfindungsgemäßen Bakteriorhodopsin-Modifikationen wird ferner ein Verfahren vorgesehen, bei dem man

(a) Halobacterium spec. mit funktionell intaktem Bakteriorhodopsin einer Mutagenese in an sich bekannter Weise unterwirft,

(b) das erhaltene Zellgemisch unter anaeroben Bedingungen in Gegenwart von 5-Brom-2'-deoxyuridin (BDU) in an sich bekannter Weise so lange kultiviert, bis eine ohne BDU kultivierte Kontrollkultur die späte logarithmische oder stationäre Phase erreicht,

(c) das in Gegenwart von BDU kultivierte Zellgemisch mit Licht einer Wellenlänge im Bereich von 300 bis 550 nm in an sich bekannter Weise belichtet, um nicht mutierte Zellen abzutöten, und

(d) gegebenenfalls die Stufen (b) und (c) wiederholt, wobei das Verfahren dadurch gekennzeichnet ist, daß man

(I) bei der Stufe (a) von einem Wildtyp mit einer Reversionsrate kleiner 1:10⁶ ausgeht,

(II) die Belichtung der Stufe (c) mit 100 bis 200 mW/cm² 5 min bis 2 h lang durchführt,

(III) die Kultivierung der Stufe (b) in Gegenwart von Retinal durchführt oder mit dem aus Stufe (c) oder (d) erhaltenen Zellgemisch die Stufe (b) in Gegenwart von Retinal und danach die stufe (c) und gegebenenfalls die Stufe (d) wiederholt, um retinalnegative Mutanten abzutöten,

(IV) das aus Stufe (III) erhaltene Zellgemisch aerob als Plattenkultur anzüchtet,

(V) die angezüchtete Plattenkultur anaerob weiterkultiviert,

(VI) alle Klone, die anaerob phototroph nicht mehr wachsen, wie Klone mit Bakterio-opsin-Modifikationen oder mit gestörter Bakterio-opsin-Synthese, mit Antikörpern gegen den C-Terminus der Bakterio-opsin-Modifikationen (bzw. Bakteriorhodopsin-Modifikationen) einem Kolonie-Screening in an sich bekannter Weise unterwirft und

(VII) Klone die Bakteriorhodopsin-Modifikationen exprimieren, in an sich bekannter Weise aerob kultiviert und in an sich bekannter Weise die Bakteriorhodopsin-Modifikationen gewinnt.

Vorzugsweise führt man die Belichtung der Stufe (c) mit 100 bis 200 mW/cm² 20 min bis 1 h lang durch.

Es hat sich überraschenderweise gezeigt, daß durch die Verfahrensstufe (V) Mutanten, die Bakteriorhodopsin (nur noch) schwach exprimieren, derart aktiviert werden, daß sie bei der folgenden Stufe (VI) wachsen (was sie ohne die Anzüchtung nicht tun würden), so daß sie von anaerob phototroph nicht mehr wachsenden Mutanten unterschieden und abgetrennt werden können.

Gemäß einer weiteren Ausführungsform der Erfindung kann man die genannten Bakteriorhodopsin-Modifikationen auch dadurch herstellen, daß man einen erfindungsgemäßen Halobacterium-Stamm kultiviert, eine erfindungsgemäße Bakteriorhodopsin-Modifikation exprimieren läßt und diese Modifikation gewinnt.

Ein Beispiel für eine Halobacterium species ist Halobacterium halobium.

Nachstehend wird die Erfindung beispielhaft näher erläutert.

UV-Mutagenese

Eine Kultur (37 ml; $10^9$ Zellen/ml) von Wildzellen (aus einem südfranzösischen Salztümpel) wurde bis zur späten logarithmischen Phase kultiviert. Die Zellen wurden abzentrifugiert und in 10 ml Basalsalz resuspendiert. Die Suspension wurde in offene Petrischalen gegossen und von oben mit Licht einer Wellenlänge von 254 nm (UV-Lampe von Desaga) belichtet. Bei einer Belichtungsdauer von 5 min betrug die Überlebensrate 0,2 %.

BDU-Selektion

35 ml Medium wurden mit 4 ml der mutagenisierten Zellsuspension gemischt und bis zur späten logarithmischen Phase kultiviert. 4 ml dieser Kultur wurden unter anaeroben Bedingungen eine Woche lang in einem Medium kultiviert, das 17,5 mg BDU und 35 mg Uridin in 35 ml enthielt. Während dieser Zeit erreichte eine Kontrollkultur ohne BDU/Uridin die stationäre Phase. Die Zellsuspension wurde zentrifugiert, in 10 ml Basalsalz resuspendiert und in eine Petrischale gegossen, die auf einem eisgekühlten Metallblock stand. Danach wurde die Suspension mit einer 300-Watt-Lampe (Philips MLU) von oben 1 h lang belichtet. Das Licht wurde durch ein BG3-Glas (Schott) und den Plastikdeckel der Petrischale gefiltert. Auf diese Weise wurde ein Wellenlängenbereich von 300 bis 460 nm selektiert. Die Bestrahlungsstärke betrug 150 mW/cm$^2$ bei einer Entfernung der Lampe zur Schale von 20 cm. Nach der Belichtung ließ man die Zellen (5 ml) aerob bis zur späten logarithmischen Phase wachsen.

Das genannte Anreicherungsverfahren mit BDU selektiert solche Zellen, die die Fähigkeit verloren haben, unter anaeroben Bedingungen phototroph zu wachsen. Da dieses Wachstum von Bakteriorhodopsin vermittelt wird, umfaßte das erhaltene Zellgemisch folgende Mutantentypen:

(1) Mutanten mit verändertem Bakteriorhodopsin-Strukturgen (erwünscht);

(2) retinalnegative Mutanten und

(3) Mutanten mit gestörter Retinalsynthese (im vorliegenden Zusammenhang unerwünscht); sowie

(4) Mutanten mit gestörter Bakterio-opsin-Regulation (im vorliegenden Zusammenhang unerwünscht).

Nach den geschilderten Maßnahmen wurde Retinal zum Wachstumsmedium zugegeben und das BDU-Selektionsverfahren wiederholt. Da sich die Mutanten gemäß (2) und (3) in Gegewart von Retinal wie der Wildtyp verhalten, also wachsen und BDU inkorporieren, wurden sie bei der geschilderten weiteren Stufe abgetötet. Die Überlebensrate in eine Wildtypkultur betrug unter diesen Bedingungen $10^{-4}$.

Klonieren und Antikörper-Screening

Die verbliebenen Mutanten wurden auf folgende Weise charakterisiert. Nach Vereinzelung wurden Klone auf Agarplatten aerob angezüchtet, wonach der Hauptteil der Zellmasse auf neue Agarplatten übertragen und anaerob im Licht inkubiert wurde, um solche Zelltypen zu ermitteln bzw. auszuschließen, die noch anaerob wachsen konnten. Alle Klone, die anaerob nicht mehr phototroph wuchsen, wurden einem Kolonie-Screening in Hinblick auf Bakteriorhodopsin-Genexpression unterzogen. Dazu wurden die Zellen nach aerobem Wachstum von einer Agarplatte auf Nitrozellulosefilter übertragen. Mit Whatman-3MM-Papier wurde die überschüssige Zellmasse entfernt. Die Zellen wurden durch dreimaliges Waschen mit einer kochenden 0,1-proz. SDS-Lösung (Natriumdodecylsulfat) in TBS (10 mM Tris/HCl pH 7,5, 0,15 M NaCl) aufgeschlossen und die Filter danach mit heißem TBS gewaschen. Alle weiteren Inkubationen mit Ausnahme der abschließenden Farbreaktion fanden in einer Lösung von 0,05 % Tween 20 in TBS statt. Die Filter wurden je zwei Stunden mit einem monoklonalen Antikörper gegen den C-Terminus von Bakterioopsin und mit einem peroxydasegekoppelten Antikörper, der gegen den ersten Antikörper gerichtet war, inkubiert. Vor, nach und zwischen diesen Inkubationen wurden sie je 30 Minuten bei mehrmaligem Wechseln der Lösung gewaschen. Positive Klone wurden dann durch eine 10- bis 20-minütige Inkubation in einer Substratlösung (3 mg/ml Chlornaphtol in Methanol + 5 Vol. TBS, 0,01 % Wasserstoffperoxyd) identifiziert.

4

| Mutante lfd. Nr. | Wellenlänge maximaler Absorption | Lebenszeit des langlebigsten Intermediats msec. |
|---|---|---|
| 384 | 610 | |
| 413 | 550 | |
| 350 | 510 | |
| 372 | 490 | |
| 374 | | 80 |
| 369 | | 450 |
| 412 | | 520 |
| 326 | | 700 |
| 351 | | 980 |
| Wildtyp (zum Vergleich) | 560 | 10 |

**Patentansprüche**

1. Halobacterium-Stämme, die Bakteriorhodopsin-Modifikationen
   - mit einer gegenüber dem Bakteriorhodopsin des Wildtyps verlängerten Lebenszeit des langlebigsten Zwischenprodukts von mehr als 10 msec und/oder
   - mit einer Farbe im Bereich von 400 bis 650 nm produzieren, wobei die Farbe des Wildtyps von 560 nm ausgenommen ist.

2. Verfahren zur Gewinnung von Halobacterium-Stämmen gemäß Anspruch 1, bei dem man
   (a) Halobacterium spec. mit funktionell intaktem Bakteriorhodopsin einer Mutagenese in an sich bekannter Weise unterwirft,
   (b) das erhaltene Zellgemisch unter anaeroben Bedingungen in Gegenwart von 5-Brom-2'-deoxyuridin (BDU) in an sich bekannter Weise so lange kultiviert, bis eine ohne BDU kultivierte Kontrollkultur die späte logarithmische oder stationäre Phase erreicht,
   (c) das in Gegenwart von BDU kultivierte Zellmaterial mit Licht einer Wellenlänge im Bereich von 300 bis 550 nm in an sich bekannter Weise belichtet, um nicht mutierte Zellen abzutöten, und
   (d) gegebenenfalls die Stufen (b) und (c) wiederholt, dadurch **gekennzeichnet,** daß man
   (I) bei der Stufe (a) von einem Wildtyp mit einer Reversionsrate kleiner 1:10$^6$ ausgeht,
   (II) die Belichtung der Stufe (c) mit 100 bis 200 mW/cm$^2$ 5 min bis 2 h lang durchführt,
   (III) die Kultivierung der Stufe (b) in Gegenwart von Retinal durchführt oder mit dem aus Stufe (c) oder (d) erhaltenen Zellgemisch die Stufe (b) in Gegenwart von Retinal und danach die Stufe (c) und gegebenenfalls (d) wiederholt, um retinalnegative Mutanten abzutöten,
   (IV) das aus Stufe (III) erhaltene Zellgemisch aerob als Plattenkultur anzüchtet,
   (V) die angezüchtete Plattenkultur anaerob weiterkultiviert, (VI) alle Klone, die anaerob phototroph nicht mehr wachsen mit Antikörpern gegen den C-Terminus der Bakterio-opsin-Modifikationen einem Kolonie-Screening in an sich bekannter Weise unterwirft und
   (VII) Klone, die Bakteriorhodopsin-Modifikationen exprimieren, in an sich bekannter Weise gewinnt.

3. Bakteriorhodopsin-Modifikation mit einer gegenüber dem Bakteriorhodopsin eines Halobacterium-Wildtyps verlängerter Lebenszeit des langlebigsten Zwischenprodukts von mehr als 10 msec.

4. Bakteriorhodopsin-Modifikation nach Anspruch 3, **gekennzeichnet** durch eine Lebenszeit des langlebigsten Zwischenprodukts im Bereich von mehr als 10 msec. bis 1 sec. oder 1 min oder unendlich, insbesondere im Bereich von 0,5 sec bis unendlich.

5. Verfahren zur Gewinnung von Bakteriorhodopsin-Modifikationen gemäß einem der Ansprüche 3 bis 5, bei dem man

(a) Halobacterium spec. mit funktionell intaktem Bakteriorhodopsin einer Mutagenese in an sich bekannter Weise unterwirft,

(b) das erhaltene Zellgemisch unter anaeroben Bedingungen in Gegenwart von 5-Brom-2'-deoxyuridin (BDU) in an sich bekannter Weise so lange kultiviert, bis eine ohne BDU kultivierte Kontrollkultur die späte logarithmische oder stationäre Phase erreicht,

(c) das in Gegenwart von BDU kultivierte Zellmaterial mit Licht einer Wellenlänge im Bereich von 300 bis 550 nm in an sich bekannter Weise belichtet, um nicht mutierte Zellen abzutöten, und

(d) gegebenenfalls die Stufen (b) und (c) wiederholt, dadurch **gekennzeichnet,** daß man

(I) bei der Stufe (a) von einem Wildtyp mit einer Reversionsrate kleiner 1:10$^6$ ausgeht,

(II) die Belichtung der Stufe (c) mit 100 bis 200 mW/cm$^2$ 5 min bis 2 h lang durchführt,

(III) die Kultivierung der Stufe (b) in Gegenwart von Retinal durchführt oder mit dem aus Stufe (c) oder (d) erhaltenen Zellgemisch die Stufe (b) in Gegenwart von Retinal und danach die Stufe (c) und gegebenenfalls (d) wiederholt, um retinalnegative Mutanten abzutöten,

(IV) das aus Stufe (III) erhaltene Zellgemisch aerob als Plattenkultur anzüchtet,

(V) die angezüchtete Plattenkultur anaerob weiterkultiviert,

(VI) alle Klone, die anaerob phototroph nicht mehr wachsen mit Antikörpern gegen den C-Terminus der Bakterio-opsin-Modifikationen einem Kolonie-Screening in an sich bekannter Weise unterwirft und

(VII) Klone, die Bakteriorhodopsin-Modifikationen exprimieren, in an sich bekannter Weise aerob kultiviert und in an sich bekannter Weise die Bakteriorhodopsin-Modifikationen gewinnt.

6. Verfahren nach Anspruch 5, dadurch **gekennzeichnet,** daß man bei der Stufe (c) 20 min bis 1 h lang belichtet.

7. Verfahren zur Gewinnung einer Bakteriorhodopsin-Modifikation gemäß einem der Ansprüche 3 bis 5, dadurch **gekennzeichnet,** daß man einen Halobacterium-Stamm gemäß Anspruch 1 kultiviert, die Bakteriorhodopsin-Modifikation exprimieren läßt und die exprimierte Bakteriorhodopsin-Modifikation gewinnt.

**Claims**

1. Halobacterium strains which produce bacteriorhodopsin modifications
   - with an increased life of the intermediate with the longest life, of more than 10 msec, compared with the wild-type bacteriorhodopsin and/or
   - with a colour in the range from 400 to 650 nm, excluding the colour of the wild type of 560 nm.

2. Process for isolating Halobacterium strains according to Claim 1, in which
   (a) Halobacterium spec. with functionally intact bacteriorhodopsin is subjected to a mutagenesis in a manner known per se,
   (b) the resulting cell mixture is cultivated under anaerobic conditions in the presence of 5-bromo-2'-deoxyuridine (BDU) in a manner known per se until a control culture cultivated without BDU reaches the late logarithmic or stationary phase,
   (c) the cell material cultivated in the presence of BDU is irradiated with light of a wavelength in the range from 300 to 550 nm in a manner known per se in order to kill non-mutated cells, and
   (d) where appropriate Stages (b) and (c) are repeated, characterised in that
       (I) Stage (a) starts with a wild type with a reversion rate less than 1:10$^6$,
       (II) the irradiation in Stage (c) is carried out with 100 to 200 mW/cm$^2$ for 5 min to 2 h,
       (III) the cultivation in Stage (b) is carried out in the presence of retinal, or the cell mixture obtained from Stage (c) or (d) is subjected to repetition of Stage (b) in the presence of retinal and then of Stage (c) and, where appropriate, Stage (d) in order to kill retinal-negative mutants,
       (IV) the cell mixture obtained from Stage (III) is initially cultured aerobically as plate culture,
       (V) the initially cultured plate culture is further cultivated anaerobically,
       (VI) all clones which no longer show anaerobic phototrophic growth are subjected to a colony screening in a manner known per se with antibodies against the C terminus of the bacterioopsin modifications, and
       (VII) clones which express bacteriorhodopsin modifications are isolated in a manner known per se.

3. Bacteriorhodopsin modification with an increased life of the intermediate with the longest life, of more than 10 msec, compared with the wild-type bacteriorhodopsin.

4. Bacteriorhodopsin modification according to Claim 3, characterised by a life of the intermediate with the longest life in the range from more than 10 msec to 1 sec or 1 min or infinity, in particular in the range from 0.5 sec to infinity.

5. Process for isolating bacteriorhodopsin modifications according to either of Claims 3 or 4, in which
(a) Halobacterium spec. with functionally intact bacteriorhodopsin is subjected to a mutagenesis in a manner known per se,
(b) the resulting cell mixture is cultivated under anaerobic conditions in the presence of 5-bromo-2'-deoxyuridine (BDU) in a manner known per se until a control culture cultivated without BDU reaches the late logarithmic or stationary phase,
(c) the cell material cultivated in the presence of BDU is irradiated with light of a wavelength in the range from 300 to 550 nm in a manner known per se in order to kill non-mutated cells, and
(d) where appropriate Stages (b) and (c) are repeated, characterised in that
(I) Stage (a) starts with a wild type with a reversion rate less than $1:10^6$,
(II) the irradiation in Stage (c) is carried out with 100 to 200 $mW/cm^2$ for 5 min to 2 h,
(III) the cultivation in Stage (b) is carried out in the presence of retinal, or the cell mixture obtained from Stage (c) or (d) is subjected to repetition of Stage (b) in the presence of retinal and then of Stage (c) and, where appropriate, Stage (d) in order to kill retinal-negative mutants,
(IV) the cell mixture obtained from Stage (III) is initially cultured aerobically as plate culture,
(V) the initially cultured plate culture is further cultivated anaerobically,
(VI) all clones which no longer show anaerobic phototrophic growth are subjected to a colony screening in a manner known per se with antibodies against the C terminus of the bacterioopsin modifications, and
(VII) clones which express bacteriorhodopsin modifications are cultivated aerobically in a manner known per se, and the bacteriorhodopsin modifications are isolated in a manner known per se.

6. Process according to Claim 5, characterised in that irradiation is carried out in Stage (c) for 20 min to 1 h.

7. Process for isolating a bacteriorhodopsin modification according to any of Claims 3 to 5, characterised in that a Halobacterium strain according to Claim 1 is cultivated, the bacteriorhodopsin modification is expressed, and the expressed bacteriorhodopsin modification is isolated.

## Revendications

1. Souches d'Halobacterium qui produisent des modifications de bactériorhodopsine
   - avec, par rapport à la bactériorhodopsine de type sauvage, une durée de vie prolongée du produit intermédiaire ayant la plus longue durée de vie, de plus de 10 msec, et/ou
   - avec une couleur qui correspond à l'intervalle de 400 à 650 nm,
   la couleur du type sauvage à 560 nm étant exclue.

2. Procédé pour obtenir des souches d'Halobacterium selon la revendication 1, dans lequel :
   (a) on soumet à une mutagenèse, d'une manière en elle-même connue, Halobacterium spec. à bactériorhodopsine fonctionnement intacte,
   (b) de la manière en elle-même connue on cultive le mélange de cellules obtenu dans des conditions anaérobies, en présence de 5-bromo-2'-désoxyuridine (BDU), jusqu'à ce qu'une culture témoin cultivée sans BDU ait atteint la phase retard logarithmique ou stationnaire,
   (c) on irradie de manière connue le mélange de cellules cultivées en présence de BDU par un rayonnement d'une longueur d'onde de 300 à 550 nm pour détruire les cellules qui n'ont pas muté, et
   (d) on recommence éventuellement les opérations (b) et (c), procédé caractérisé en ce que :
      (I) on effectue l'opération (a) avec un type sauvage à un rapport d'inversion inférieur à $1:10^6$,
      (II) on effectue l'irradiation (c) à 100 à 200 $mW/cm^2$ pendant une durée de 5 minutes à 2 heures,
      (III) on effectue la culture (b) en présence de rétinal ou bien avec le mélange de cellules obtenu en (c) ou (d) en présence de rétinal puis on recommence l'opération (c) et le cas échéant

7

l'opération (d), pour détruire les mutants rétinalnégatifs,

(IV) on cultive en culture sur plaque par voie aérobie le mélange de cellules provenant de l'opération III,

(V) on poursuit la culture III par voie anaérobie,

(VI) on soumet de manière connue à une sélection de colonnies tous les clones qui ne se multiplient plus en phototropisme anaérobie avec des anti-corps contre l'extrémité C des modifications de la bactério-opsine, et

(VII) on récupère de manière en elle-même connue les clones qui expriment les modifications de bactériorhodopsine

3. Modifications de bactériorhodopsine avec, par rapport à la bactériorhodopsine d'un type sauvage d'Halobacterium, une durée de vie prolongée du produit intermédiaire à vie la plus longue, de plus de 10 msec.

4. Modifications de bactériorhodopsine selon la revendication 3, caractérisées par une durée de vie du produit intermédiaire à vie la plus longue de plus de 10 msec jusqu'à 1 seconde ou 1 minute ou jusqu'à l'infini, notamment entre 0,5 seconde et l'infini.

5. Procédé pour obtenir des modifications de bactériorhodopsine selon l'une des revendications 3 et 4, procédé dans lequel :

(a) on soumet de manière en elle-même connue à une mutagenèse Halobacterium spec. à bactériorhodopsine fonctionnellement intacte,

(b) on cultive de manière connue le mélange de cellules obtenu dans des conditions anaérobies en présence de 5-bromo-2'-désoxyuridine (BDU) jusqu'à ce qu'une culture témoin cultivée sans BDU ait atteint la phase retard logarithmique ou stationnaire,

(c) on irradie le mélange de cellules cultivées en présence de BDU avec un rayonnement d'une longueur d'onde de 300 à 550 nm pour détruire les cellules qui n'ont pas muté, et

(d) le cas échéant on recommence les opérations (b) et (c), procédé caractérisé en ce que :

(I) on effectue l'opération (a) avec un type sauvage à un rapport d'inversion inférieur à $1:10^6$,

(II) on effectue l'irradiation (c) à 100 à 200 mW/cm$^2$ pendant une durée de 5 minutes à 2 heures,

(III) on effectue la culture (b) en présence de rétinal ou bien avec le mélange de cellules provenant de l'opération (c) ou (d) en présence de rétinal puis on recommence l'opération (c) et le cas échéant l'opération (d), pour détruire les mutants rétinalnégatifs,

(IV) on cultive par voie aérobie en culture sur plaque le mélange de cellules provenant de l'opération III,

(V) on poursuit la culture en plaque par voie anaérobie,

(VI) on soumet de manière connue à une sélection de colonies tous les clones qui ne se multiplient plus en phototropisme anaérobie avec des anti-corps contre l'extrémité C des modifications de bactérioopsine, et

(VII) on cultive de manière connue par voie aérobie les clones qui expriment les modifications de bactériorhodopsine, et on récupère de manière connue les modifications de bactériorhodopsine.

6. Procédé selon la revendication 5, caractérisé en ce que dans l'opération (c) on effectue l'irradiation pendant une durée de 20 minutes à 1 heure.

7. Procédé pour obtenir une modification du bactériorhodopsine selon l'une des revendications 3 à 5, procédé caractérisé en ce que l'on cultive une souche d'Halobacterium selon la revendication 1, on laisse exprimer la modification de bactériorhodopsine et on récupère la modification exprimée.